# EUROPEAN PATENT APPLICATION

(11) **EP 4 371 529 A1**
(43) Date of publication of application: **22.05.2024**
(21) Application number: 23208241.2
(22) Date of filing: 07.11.2023
(51) Int. Cl.: A61F 2/24

(54) **VALVE PROSTHESIS WITH PROSTHETIC VALVE HAVING DURABLE COMMISSURES**

(30) Priority: 15.11.2022 US 202263425501 P; 23.10.2023 US 202318492557
(71) Applicant: Medtronic Vascular, Inc., Santa Rosa, CA 95403 (US)
(72) Inventor: Dorman, Kyle J., Santa Ana, 92705 (US); Olney, Karl L., Santa Ana, 92705 (US); Cheshko, Tasha, Santa Ana, 92705 (US); Goshgarian, Justin G., Santa Rosa, 95403 (US); Kibria, Alkindi, Santa Ana, 92705 (US)
(74) Representative: Zimmermann & Partner Patentanwälte mbB

(57) **Abstract**

The techniques of this disclosure generally relate to a valve prosthesis including a one-piece molded valve comprising commissures and reinforcement members applied to the commissures. The reinforcement members provide reinforcement and strength to the commissures, e.g., from the stress applied by commissure stitching on the commissures.

## Description

### CROSS-REFERENCE TO RELATED APPLICATIONS

This application claims the benefit of U.S. Provisional Application No. 63/425,501, filed November 15, 2022, the contents of which are incorporated by reference herein in their entirety.

### FIELD

The present technology is generally related to transcatheter heart valves.

### BACKGROUND

A human heart includes four heart valves that determine the pathway of blood flow through the heart: the mitral valve, the tricuspid valve, the aortic valve, and the pulmonary valve. The mitral and tricuspid valves are atrioventricular valves, which are between the atria and the ventricles, while the aortic and pulmonary valves are semilunar valves, which are in the arteries leaving the heart. Ideally, native leaflets of a heart valve move apart from each other when the valve is in an open position, and meet or "coapt" when the valve is in a closed position. Problems that may develop with valves include stenosis in which a valve does not open properly, and/or insufficiency or regurgitation in which a valve does not close properly. Stenosis and insufficiency may occur concomitantly in the same valve. The effects of valvular dysfunction vary, with regurgitation or backflow typically having relatively severe physiological consequences to the patient.

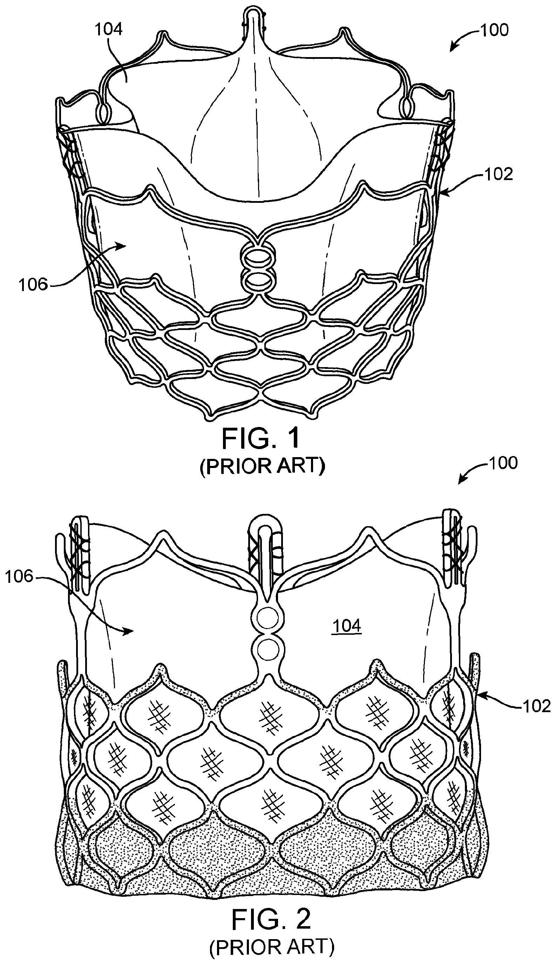

Recently, flexible prosthetic valves supported by stent structures that can be delivered percutaneously using a catheter-based delivery system have been developed for heart and venous valve replacement. FIG. 1 is a perspective view of a valve prosthesis 100 in accordance with the prior art. FIG. 2 is a side view of valve prosthesis 100 of FIG. 1 in accordance with the prior art. Referring to FIGS. 1 and 2, valve prosthesis 100 may include a balloon-expandable stent structure 102 with valve leaflets 104 attached to the interior of stent structure 102. Valve leaflets 104 may be parts of a 3D single piece prosthetic valve 106.

Valve prosthesis 100 can be reduced in diameter, by crimping onto a balloon catheter, and advanced through the venous or arterial vasculature. Once valve prosthesis 100 is positioned at the treatment site, for instance within an incompetent native valve, stent structure 102 may be expanded to hold valve prosthesis 100 firmly in place.

When designing a valve prosthesis such as valve prosthesis 100, valve-frame integration and frame mechanical performance often have competing needs or requirements. For example, when attaching the valve to the frame during valve-frame integration, the valve itself needs to be reinforced to the frame at certain locations without hindering mechanical performance of the frame. Embodiments hereof relate to an improved balloon-expandable transcatheter valve prosthesis configured to minimize tradeoffs between the above-described competing needs.

### SUMMARY

The techniques of this disclosure generally relate to a valve prosthesis including a one-piece molded valve comprising commissures and reinforcement members applied to the commissures.

In one aspect, the present disclosure provides a valve prosthesis including a one-piece molded valve comprising commissures and commissure cuts in the commissures.

In another aspect, the present disclosure provides a valve prosthesis including a one-piece molded valve. The one-piece molded valve includes commissures and commissure tabs extending radially outward from the commissures. Each commissure tab includes a radially extending body, a first ears attached to the body, and a second ear attached to the body.

In another aspect, the present disclosure provides a method including providing a frustum shaped tube having a folding pattern in a commissure region. The method further includes bringing free edges of valve leaflets together to cause the folding pattern to protrude outward, the folding pattern comprising a first folding line, a second folding line, a third folding line, and a fourth folding line. The second folding line and the third folding line are pulled apart from the first folding line and the fourth folding line to form a commissure tab as a T-shaped protrusion.

In another aspect, the present disclosure provides a valve prosthesis including a one-piece molded valve. The one-piece molded valve includes a commissure and a commissure tab extending radially outward from the commissure. The commissure tab includes a main body, a first ear extending perpendicularly from the main body, and a second ear extending perpendicularly from the main body and in a direction opposite the first ear.

In another aspect, the present disclosure provides a valve prosthesis including a stent structure including a trident post having a Y-shape. The trident post includes a main trunk, a first branch extending distally from the main trunk, and a second branch extending distally from the main trunk.

Further disclosed herein is a valve prosthesis including a one-piece molded valve comprising commissures and reinforcement members applied to the commissures, wherein the reinforcement members provide reinforcement and strength to the commissures, e.g., from the stress applied by commissure stitching on the commissures.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a perspective view of a valve prosthesis in accordance with the prior art.
FIG. 2 is a side view of the valve prosthesis of FIG. 1 in accordance with the prior art.
FIG. 3 is a perspective view of a valve prosthesis in an expanded configuration in accordance with one embodiment.
FIG. 4 is an outflow end view of a prosthetic valve of the valve prosthesis of FIG. 3 in accordance with one embodiment.
FIG. 5 is a perspective view of the prosthetic valve including a reinforcement member of the valve prosthesis of FIG. 3 in accordance with one embodiment.
FIG. 6 is a side view of the valve prosthesis of FIG. 3 in accordance with one embodiment.
FIG. 7 is a laid flat plan view of a tissue coupon illustrating the prosthetic valve including the reinforcement members of FIGS. 3-6 in accordance with one embodiment.
FIG. 8 is a laid flat plan view of a tissue coupon illustrating a prosthetic valve including commissure cuts in accordance with another embodiment.
FIG. 9 is a perspective view of the prosthetic valve of FIG. 8 including commissure tabs in accordance with one embodiment.
FIG. 10 is an enlarged view of a region X of the prosthetic valve including a commissure tab of FIG. 9 in accordance with one embodiment.
FIG. 11 is a top plan view of a commissure and the commissure tab of FIG. 10, and a trident post in accordance with one embodiment.
FIG. 12 is a top plan view of the commissure and the commissure tab of FIG. 10, and the trident post in accordance with another embodiment.
FIG. 13 is a laid flat plan view of a tissue coupon illustrating a prosthetic valve in accordance with another embodiment.
FIG. 14 is enlarged plan view of a region XIV of the prosthetic valve of FIG. 13 illustrating a folding pattern of a commissure tab in accordance with one embodiment.
FIG. 15 is a top plan view of the commissure tab being attached around a trident post in accordance with one embodiment.
FIG. 16 is a top plan view of the commissure tab attached to the trident post of FIG. 15 in accordance with one embodiment.
FIG. 17 is a laid flat plan view of a tissue coupon illustrating a prosthetic valve in accordance with another embodiment.
FIG. 18 is side view of a commissure tab of the prosthetic valve of FIG. 17 attached to a trident post in accordance with one embodiment.
FIG. 19 is an inner perspective view of the commissure tab of the prosthetic valve of FIG. 17 attached to the trident post of FIG. 18 in accordance with one embodiment.
FIG. 20 is an outer perspective view of the commissure tab of the prosthetic valve of FIG. 17 attached to the trident post of FIG. 18 in accordance with one embodiment.
FIG. 21 is a perspective view of a Y-shaped trident post in accordance with one embodiment.
FIG. 22 is a perspective view of a commissure tab attached to the trident post of FIG. 21 in accordance with one embodiment.
FIG. 23 is a top plan view of the commissure tab attached to the trident post of FIG. 22 in accordance with one embodiment.
FIG. 24 is a side view of a valve prosthesis in accordance with another embodiment.

### DETAILED DESCRIPTION

Specific embodiments are now described with reference to the figures, wherein like reference numbers indicate identical or functionally similar elements. The terms "distal" and "proximal", when used in the following description to refer to a native vessel, native valve, or a device to be implanted into a native vessel or native valve, such as a heart valve prosthesis, are with reference to the direction of blood flow. Thus, "distal" and "distally", also referred to as the "outflow" or "outflow direction", respectively, refer to positions in a downstream direction with respect to the direction of blood flow. The terms "proximal" and "proximally", also referred to as the "inflow" or "inflow direction", respectively, refer to positions in an upstream direction with respect to the direction of blood flow.

Although the description is in the context of treatment of an aortic heart valve, embodiments may also be used where it is deemed useful in other valved intraluminal sites that are not in the heart. For example, embodiments may be applied to other heart valves or venous valves as well.

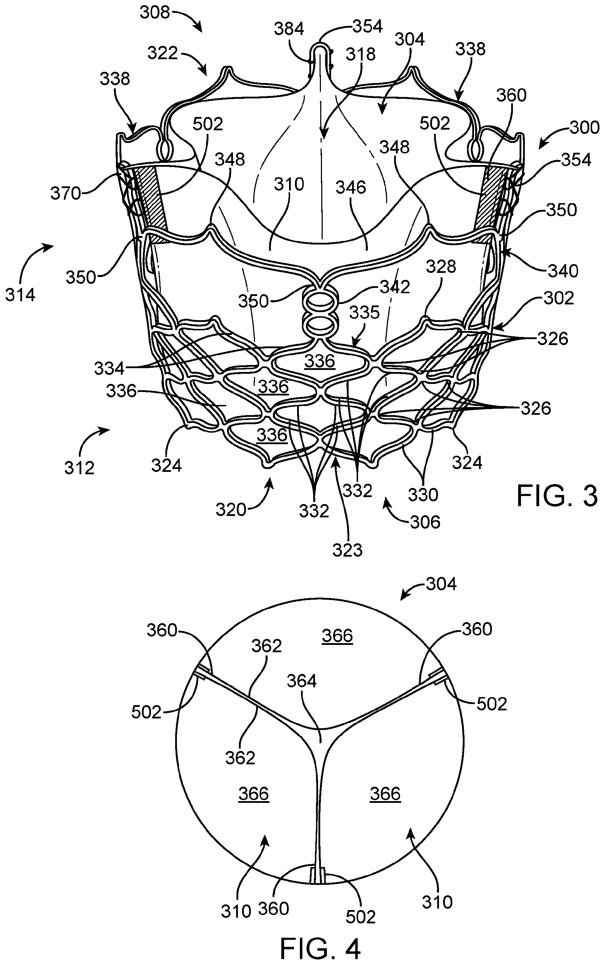

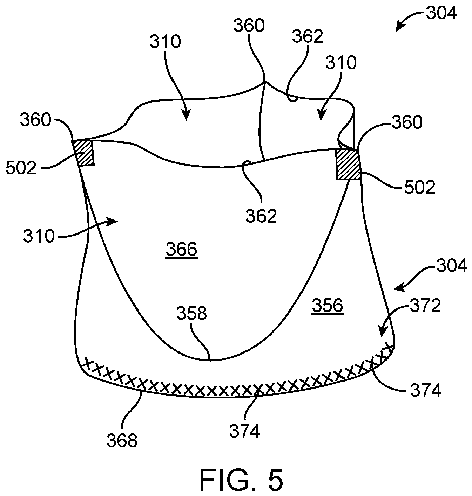

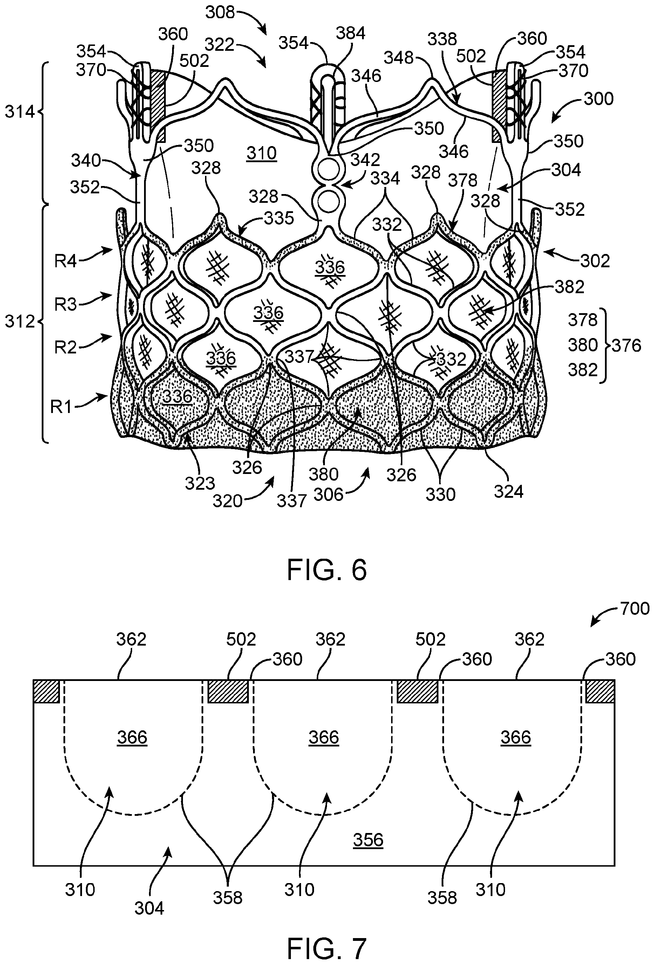

FIG. 3 is a perspective view of a transcatheter valve prosthesis 300 in an expanded configuration in accordance with one embodiment. FIG. 4 is an outflow end view of a prosthetic valve 304 of valve prosthesis 300 of FIG. 3 in accordance with one embodiment. FIG. 5 is a perspective view of prosthetic valve 304 including reinforcement members 502 of valve prosthesis 300 of FIG. 3 in accordance with one embodiment. FIG. 6 is a side view of valve prosthesis 300 of FIG. 3 in accordance with one embodiment. FIG. 4 illustrates prosthetic valve 304 in a closed state while FIGS. 3, 5 and 6 illustrates prosthetic valve 304 in an open state.

Referring now to FIGS. 3-6 together, valve prosthesis 300 has a radially-expandable stent structure 302, sometimes called the frame or frame structure, and prosthetic valve 304. Stent structure 302 is generally tubular, and is mechanically or balloon expandable, having a crimped configuration for delivery within a vasculature and an expanded configuration for deployment within a native heart valve. When valve prosthesis 300 is deployed within the valve annulus of a native heart valve, stent structure 302 of valve prosthesis 300 is configured to be radially expanded within native valve leaflets of the patient's defective valve, to thereby retain the native valve leaflets in a permanently open state. In embodiments hereof, valve prosthesis 300 is configured for replacement of an aortic valve such that an inflow end 306, i.e., the proximal end, of valve prosthesis 300 extends into and anchors within the aortic annulus of a patient's left ventricle, while an outflow end 308, i.e., the distal end, of valve prosthesis 300 is positioned within the aortic sinuses.

Stent structure 302 of valve prosthesis 300 may be a unitary frame or scaffold that supports prosthetic valve 304 including one or more valve leaflets 310 within the interior of stent structure 302. Prosthetic valve 304 is capable of blocking flow in one direction to regulate flow there-through via valve leaflets 310 that may form a bicuspid or tricuspid replacement valve. Although prosthetic valve 304 is illustrated and discussed herein as having three valve leaflets 310, i.e., prosthetic valve 304 has a tricuspid leaflet configuration, a bicuspid leaflet configuration may be used in other embodiments. More particularly, as valve prosthesis 300 is configured for placement within a native aortic valve which typically has three leaflets, prosthetic valve 304 may include three valve leaflets 310. However, valve prosthesis 300 is not required to have the same number of leaflets as the native valve. If valve prosthesis 300 is alternatively configured for placement within a native valve having two leaflets such as the mitral valve, prosthetic valve 304 may include two or three valve leaflets 310.

Stent structure 302 is balloon-expandable. As such, stent structure 302 is made from a plastically deformable material such that when expanded by a dilatation balloon, stent structure 302 maintains its radially expanded configuration. Stent structure 302 may be formed from stainless steel such as 316L or other suitable metal, such as platinum iridium, cobalt chromium alloys such as MP35N or L605, or various types of polymers or other similar materials, including the materials coated with various surface deposits to improve clinical functionality. Stent structure 302 is configured to be rigid such that it does not deflect or move when subjected to in-vivo forces, or such that deflection or movement is minimized when subjected to in-vivo forces.

Stent structure 302 includes an inflow portion 312 and an outflow portion 314. Stent structure 302 is a tubular component defining a central lumen or passageway 318, and has an inflow end 320 and an outflow end 322. When expanded, a diameter of inflow end 320 of stent structure 302 is substantially the same as a diameter of outflow end 322 of stent structure 302 in one embodiment.

Inflow portion 312 extends distally from inflow end 320 of stent structure 302. Inflow portion 312 includes inflow crowns 324, central crowns 326, outflow crowns 328, inflow struts 330, central struts 332, and outflow struts 334. Inflow portion 312 further includes an inflow crown ring 323 defined by inflow crowns 324, inflow struts 330, and central crowns 326. Inflow portion 312 further includes an outflow crown ring 335 defined by outflow crowns 328, outflow struts 334, and central crowns 326. Inflow portion 312 generally extends between inflow crown ring 323 and outflow crown ring 335.

In between inflow crown ring 323 and outflow crown ring 335, inflow portion cells 336, sometimes called side openings, are formed in rows, and more particularly, in four rows R1, R2, R3, and R4. Each row R1, R2, R3, R4 includes 12 inflow portion cells 336. Inflow portion cells 336 of the proximal most row R1 are defined by inflow crowns 324, inflow struts 330, central crowns 326, and central struts 332. Inflow portion cells 336 of the next most proximal rows R2, R3 are defined by central crowns 326 and central struts 332. Inflow portion cells 336 of the distal most row R4 are defined by outflow crowns 328, outflow struts 334, central crowns 326, and central struts 332. Generally, inflow portion cells 336 are diamond-shaped openings having the same or identical shaped, e.g., are sometimes called symmetric.

Generally, a crown is defined where two struts connect and a node is defined as a region where two crowns connect. Accordingly, inflow crowns 324 are defined where inflow struts 330 connect. Outflow crowns 328 are defined where outflow struts 334 connect. Central crowns 326 are defined where inflow struts 330 connect, where outflow struts 334 connect, and where central struts 332 connect. Central nodes 337 are defined where central crowns 326 connect.

Outflow portion 314 is formed proximate to outflow end 322 of stent structure 302 and between outflow end 322 and inflow portion 312. Outflow portion 314 includes an outflow portion crown ring 338, commissure posts 340, and non-commissure posts 342. Outflow portion 314 can be configured in a shape that forms a central lumen or passageway.

Outflow portion crown ring 338 includes outflow portion struts 346, superior crowns 348, and inferior crowns 350. Each outflow portion struts 346 is connected at one end to an adjacent outflow crown strut 346 at a superior crown 348 and on the opposite end to an adjacent outflow crown strut 346 at an inferior crown 350. Inferior crowns 350 are connected to either a commissure post 340 or a non-commissure post 342. More particularly, every other inferior crown 350 is connected to a commissure post 340 and every other inferior crown 350 is connected to a non-commissure post 342 in an alternating repeating arrangement.

Non-commissure posts 342, sometimes called axial frame members 342, extend longitudinally between and connect inferior crowns 350 of outflow portion crown ring 338 and outflow crowns 328 of inflow portion 312. In accordance with this embodiment, non-commissure posts 342 are shaped as a figure eight and can be used as markers for depth of implant as well as clocking of valve prosthesis 300. As used herein, longitudinally is in a direction parallel with the longitudinal axis, radially is perpendicular and in a radial direction from the longitudinal axis, and circumferentially is in a plane perpendicular to the longitudinal axis and in a direction along the circumference of valve prosthesis 300.

Commissure posts 340 extend longitudinally and include axial frame members 352 and trident posts 354. Axial frame members 352 extend longitudinally between and connect inferior crowns 350 of outflow portion crown ring 338 and outflow crowns 328 of inflow portion 312. Trident posts 354 extend in a cantilever fashion and in the outflow or distal direction from inferior crowns 350 of outflow portion crown ring 338. Trident posts 354 have a central longitudinal slot 384 in this embodiment although are solid linear members in another embodiment. Axial frame members 352 and trident posts 354 are parallel with one another and are segments of commissure posts 340, which are linear members.

Prosthetic valve 304 is a one piece 3D molded structure in accordance with this embodiment. Illustratively, a single cylindrical piece is molded to form prosthetic valve 304. Accordingly, although various structures of prosthetic valve 304 are discussed below, prosthetic valve 304 is integral, i.e., formed from a single piece and not a plurality of separate pieces connected together.

Prosthetic valve 304 may be made of pericardial material; however, may instead be made of another material. Natural tissue for prosthetic valve 304 may be obtained from, for example, heart valves, aortic roots, aortic walls, aortic leaflets, pericardial tissue, such as pericardial patches, bypass grafts, blood vessels, intestinal submucosal tissue, umbilical tissue and the like from humans or animals. Synthetic materials suitable for use as prosthetic valve 304 include DACRON polyester commercially, other cloth materials, nylon blends, polymeric materials, and vacuum deposition nitinol fabricated materials. One polymeric material from which prosthetic valve 304 can be made is an ultra-high molecular weight polyethylene material. With certain materials, it may be desirable to coat one or both sides of prosthetic valve 304 with a material that will prevent or minimize overgrowth. It is further desirable that the material is durable and not subject to stretching, deforming, or fatigue.

Prosthetic valve 304 includes valve leaflets 310 and a valve inflow cylinder 356 proximal of valve leaflets 310. For example, valve inflow cylinder 356 is the remaining unmolded portion of the cylindrical material used to form prosthetic valve 304 and valve leaflets 310 are the molded portion.

Valve leaflets 310 are defined by cusps 358, commissures 360, and free edges 362. Adjoining pairs of valve leaflets 310 are attached to one another at their lateral ends to form commissures 360, with free edges 362 of valve leaflets 310 forming coaptation edges that meet in an area of coaptation 364. The region within cusps 358, commissures 360, and free edges 362 are referred to as a belly 366 of valve leaflets 310.

Valve inflow cylinder 356 has a cylindrical inflow end 368, sometimes called a nadir 368. Valve inflow cylinder 356 extends in the outflow direction from inflow end 368 as a cylinder to cusps 358, which form the outflow end of valve inflow cylinder 356. Valve inflow cylinder 356 joins valve leaflets 310 at cusps 358.

Prosthetic valve 304 is disposed within and secured to at least trident posts 354 of commissure posts 340 of stent structure 302. More particularly, commissures 360 including reinforcement members 502 are attached to trident posts 354, e.g., with commissure stitching 370. In accordance with this embodiment, trident posts 354 includes slots 384 into which commissures 360 and reinforcement members 502 are passed and then stitched using commissure stitching 370.

In addition, prosthetic valve 304 may also be disposed within and secured to inflow portion 312 of stent structure 302. More particularly, a margin of attachment (MOA) 372 of valve inflow cylinder 356, e.g., a region directly adjacent cylindrical inflow end 368, is attached to inflow portion 312, e.g., with MOA stitching 374.

Valve prosthesis 300 further includes a skirt 376, which encloses or lines a portion of stent structure 302. Skirt 376 is not illustrated in FIG. 3 for clarity of visualization of stent structure 302.

Skirt 376 may enclose or line stent structure 302. Skirt 376 may be a natural or biological material such as pericardium or another membranous tissue such as intestinal submucosa. Alternatively, skirt 376 may be a low-porosity woven fabric, such as polyester, Dacron fabric, or PTFE. In one embodiment, skirt 376 may be a knit or woven polyester, such as a polyester or PTFE knit, which can be utilized when it is desired to provide a medium for tissue ingrowth and the ability for the fabric to stretch to conform to a curved surface. Polyester velour fabrics may alternatively be used, such as when it is desired to provide a medium for tissue ingrowth on one side and a smooth surface on the other side.

In accordance with this embodiment, skirt 376 includes an outflow end outer skirt 378, an inflow end outer skirt 380, and an inner skirt 382. Outflow end outer skirt 378 is located on the outer surface of outflow crowns 328 and outflow struts 334 of inflow portion 312. Inflow end outer skirt 380 is located on the outer surface of inflow crowns 324, inflow struts 330, central crowns 326, and central struts 332. More particularly, inflow end outer skirt 380 covers first row R1 of inflow portion cells 336. Inner skirt 382 is located on the inner surface of inflow portion 312 and extends between inflow crown ring 323 and outflow crown ring 335.

FIG. 7 is a laid flat plan view of a tissue coupon 700 illustrating prosthetic valve 304 including reinforcement members 502 of FIGS. 3-6 in accordance with one embodiment. Tissue coupon 700 of FIG. 7 corresponds to prosthetic valve 304 of FIG. 5 cut longitudinally at a commissure 360. It is to be understood that tissue coupon 700 is set forth in FIG. 7 to facilitate understanding of the structure of prosthetic valve 304 and reinforcement members 502 but that prosthetic valve 304 is fabricated from a cylindrical piece of material and tissue coupon 700 would not exist during actual fabrication.

Referring now to FIGS. 3-7 together, to provide reinforcement and strength to commissures 360 of prosthetic valve 304, reinforcement members 502 are attached to prosthetic valve 304 at commissures 360.

In accordance with this embodiment, reinforcement members 502, sometimes called pledgets or additional material, are rectangular strips of material at commissures 360. Reinforcement members 502 are formed of any of the materials listed above for prosthetic valve 304 including tissue, fabric, or polymer.

Reinforcement members 502 are applied to commissures 360 using any one of a number of attachments, e.g., sutures, collagen bonding, adhesive, or other attachment techniques. In one embodiment, reinforcement members 502 are applied to commissures 360 prior to 3D molding of prosthetic valve 304.

As illustrated in FIGS. 3 and 6, commissures 360 including reinforcement members 502 are attached to trident posts 354, e.g., with commissure stitching 370. Commissure stitching 370 passes through both commissures 360 and reinforcement members 502. Accordingly, reinforcement members 502 strengthen commissures 360 from the stress applied by commissure stitching 370.

Delivery of valve prosthesis 300 may be accomplished via a percutaneous transfemoral approach or a transapical approach directly through the apex of the heart via a thoracotomy, or may be positioned within the desired area of the heart via different delivery methods known in the art for accessing heart valves. During delivery, valve prosthesis 300 remains compressed until it reaches a target diseased native heart valve, at which time a balloon of a delivery system is inflated in order to radially expand valve prosthesis 300 in situ. Valve prosthesis 300 is configured to be expanded within the native valve leaflets of the patient's defective valve, to thereby retain the native valve leaflets in a permanently open state. The delivery system is then removed and transcatheter valve prosthesis 300 remains deployed within the native target heart valve.

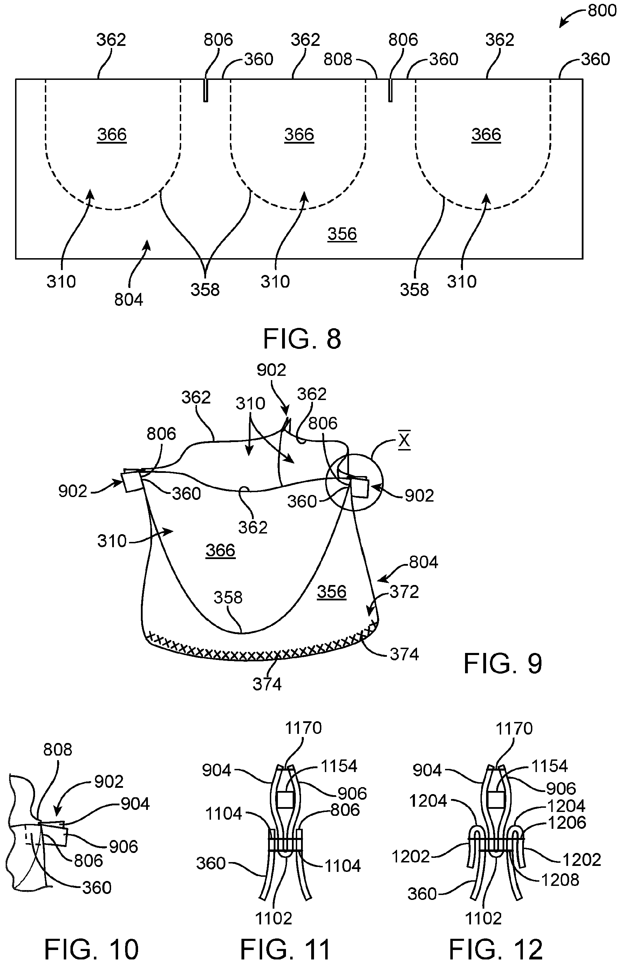

FIG. 8 is a laid flat plan view of a tissue coupon 800 illustrating a prosthetic valve 804 including commissure cuts 806 in accordance with another embodiment. FIG. 9 is a perspective view of prosthetic valve 804 of FIG. 8 including commissure tabs 902 in accordance with one embodiment. FIG. 10 is an enlarged view of a region X of prosthetic valve 804 including a commissure tab 902 of FIG. 9 in accordance with one embodiment. Tissue coupon 800 of FIG. 8 corresponds to prosthetic valve 804 of FIG. 9 cut longitudinally at a commissure 360. It is to be understood that tissue coupon 800 is set forth in FIG. 8 to facilitate understanding of the fabrication of prosthetic valve 804 and commissure tabs 902 but that prosthetic valve 804 is fabricated from a cylindrical piece of material and tissue coupon 900 would not exist during actual fabrication.

Prosthetic valve 804 of FIGS. 8-10 is similar to prosthetic valve 304 of FIGS. 3-6 and used with valve prosthesis 300 instead of prosthetic valve 304. Only the significant differences between prosthetic valve 804 of FIGS. 8-10 and prosthetic valve 304 of FIGS. 3-6 are discussed below.

Referring now to FIGS. 8-10 together, at each commissure 360, a commissure cut 806 is formed. Commissure cuts 806 are cuts in the longitudinal direction extending proximally from a distal end 808 of commissures 360. Commissure cuts 806 are formed where prosthetic valve 804 is folded to form commissures 360. Commissure cuts 806 extend proximally across the longitudinal length of commissures 360.

Commissure cuts 806 allow commissures 360 to be spread apart for insertion of commissures tabs 902 inside of commissures 360 and commissure cuts 806. Commissure tabs 902 are formed from any of the materials listed above for prosthetic valve 304 including tissue, fabric, or polymer.

FIG. 11 is a top plan view of commissure 360 and commissure tab 902 of FIG. 10, and a trident post 1154 in accordance with one embodiment. Referring now to FIGS. 8-11, in this embodiment, commissure tabs 902 are rectangular strips of material that are folded in a center 1102, inserted into commissure cuts 806, and sewn to commissures 360 with tab stitching 1104. Accordingly, each commissure tab 902 includes a first tab ear 904 and a second tab ear 906 that protrude radially outward from each center 1102 and commissure 360.

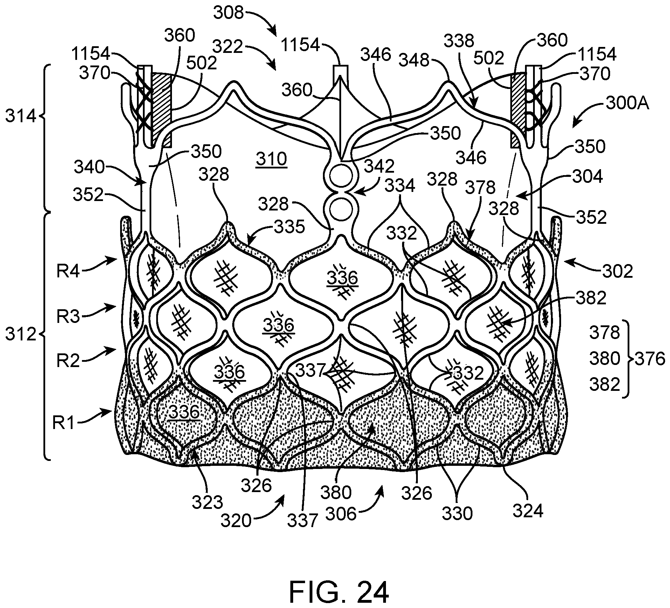

In accordance with this embodiment, trident post 1154 is a solid member in contrast to trident posts 354 of FIGS. 3, 6 that include a slot 384, see for example, FIG. 24 and the associated description below. Tab ears 904, 906 extend radially outward and around trident post 1154 and are stitched together by commissure stitching 1170. Accordingly, commissures 360 are attached to commissure tabs 902, which, in turn, are attached to trident posts 1154.

Commissure tabs 902 provide an attachment between commissures 360 and trident posts 1154. By decoupling direct attachment of commissures 360 to trident posts 1154, commissure tabs 902 reduce stress on commissures 360 and trident posts 1154.

FIG. 12 is a top plan view of commissure 360 and commissure tab 902 of FIG. 10, and trident post 1154 in accordance with another embodiment. In accordance with this embodiment, to provide strength to the connection between commissures 360 and commissure tabs 902, commissures 360, e.g., extra materials thereof, are folded back upon each other at commissure cuts 806 to form commissure reinforcement layers 1202. More particularly, commissures 360 extend radially outward to folds 1204 from which commissure reinforcement layers 1202 are folded from commissure 360 to extend radially back inward. Commissure reinforcement layers 1202 and commissures 360 are integral portions of prosthetic valve 804.

In accordance with this embodiment, commissure reinforcement layers 1202 are stitched to commissures 360 and commissure tabs 902 with reinforcement stitching 1206. Tab stitching 1208 further attaches commissures 360 to commissure tabs 902. Reinforcement stitching 1206 passes through commissure reinforcement layers 1202, commissures 360 and commissure tabs 902, whereas tab stitching 1208 passes through commissures 360 and commissure tabs 902.

Although attachment of commissure tabs 902 to trident post 1154 as solid members is illustrated and discussed above in reference to FIGS. 11 and 12, in other embodiments, commissure tabs 902 are attached inside of slots 384 of trident posts 354 of valve prosthesis 300 of FIGS. 3, 6. For example, tab ears 904, 906 are inserted into slots 384 and passed outward and around trident posts 354 and are sewn together to attach tab ears 904, 906 to trident posts 354.

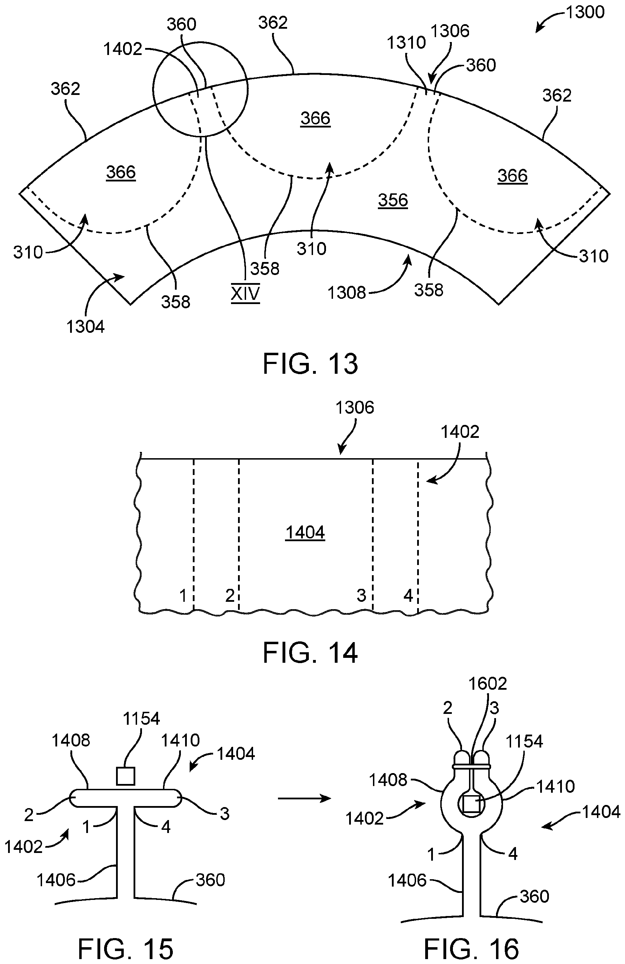

FIG. 13 is a laid flat plan view of a tissue coupon 1300 illustrating a prosthetic valve 1304 in accordance with another embodiment. FIG. 14 is enlarged plan view of a region XIV of prosthetic valve 1304 of FIG. 13 illustrating a folding pattern 1402 of a commissure tab 1404 in accordance with one embodiment. FIG. 15 is a top plan view of commissure tab 1404 being attached to a trident post 1154 in accordance with one embodiment. FIG. 16 is a top plan view of commissure tab 1404 attached to trident post 1154 of FIG. 15 in accordance with one embodiment.

Tissue coupon 1300 of FIG. 13 corresponds to prosthetic valve 1304 cut longitudinally at a commissure 360. It is to be understood that tissue coupon 1300 is set forth in FIG. 13 to facilitate understanding of the fabrication of prosthetic valve 1304 and commissure tabs 1404 but that prosthetic valve 1304 is fabricated from a frustum shaped piece of material and tissue coupon 1300 would not exist during actual fabrication.

Prosthetic valve 1304 of FIGS. 13-16 is similar to prosthetic valve 304 of FIGS. 3-6 and used with valve prosthesis 300 instead of prosthetic valve 304. Only the significant differences between prosthetic valve 1304 of FIGS. 13-16 and prosthetic valve 304 of FIGS. 3-6 are discussed below.

Referring now to FIGS. 13-16 together, tissue coupon 1300 is a non-rectangular coupon that provides extra material for folding and making of commissure tabs 1404. Illustratively, a frustum shaped tube is provided and then pinching is done to make commissure tabs 1404. Tissue coupon 1300 has a longer distal edge 1306 than a proximal edge 1308. Distal edge 1306 is sometimes called the free-edge side and proximal edge 1308 is sometimes called the MOA side.

In commissure regions 1310 between each valve leaflet 310 at distal edge 1306, tissue coupon 1300 has folding pattern 1402 including a first folding line 1, a second folding line 2, a third folding line 3, and a fourth folding line 4. Each folding line 1-4 extends proximally from distal edge 1306. Folding lines 1 and 4 are adjacent valve leaflets 310 and folding lines 2 and 3 are between folding lines 1 and 4.

As illustrated in the top plan view of FIG. 15, free edges 362 of valve leaflets 310 are brought together to cause folding pattern 1402 to protrude radially outward from commissures 360. Folding lines 2 and 3 are then pulled apart from folding lines 1 and 4 to form commissure tab 1404 as a T-shaped protrusion. Commissure tab 1404 includes a radially extending body 1406, a first ear 1408, and a second ear 1410. Ears 1408, 1410 are attached to body 1406.

As illustrated in FIG. 16, folding lines 2 and 3 are then wrapped around trident post 1154 and are stitched together with tab stitching 1602 to attach commissure tab 1404 to trident post 1154. In other words, ears 1408, 1410 are wrapped around trident post 1154 and then stitched together with tab stitching 1602 to attach the commissure tab 1404 to the respective trident post 1154.

The extra material of tissue coupon 1300 used to form commissure tabs 1404 reduces the length of distal edge 1306 to approximately equal the length of proximal edge 1308. This results in prosthetic valve 1304 having a cylindrical shape of a uniform diameter, or approximately so.

Commissure tabs 1404 provide a radially extending attachment of commissures 360 to trident posts 1154. By decoupling direct attachment of commissures 360 to trident posts 1154, commissure tabs 1404 reduce stress applied to prosthetic valve 1304.

Although attachment of commissure tabs 1404 to trident posts 1154 as solid members is illustrated and discussed above in reference to FIGS. 15 and 16, in other embodiments, commissure tabs 1404 are attached inside of slots 384 of trident posts 354 of valve prosthesis 300 of FIGS. 3, 6. For example, fold lines 2, 3 are inserted into slots 384 and passed outward and around trident posts 354 and are sewn together to attach commissure tabs 1404 to trident posts 354.

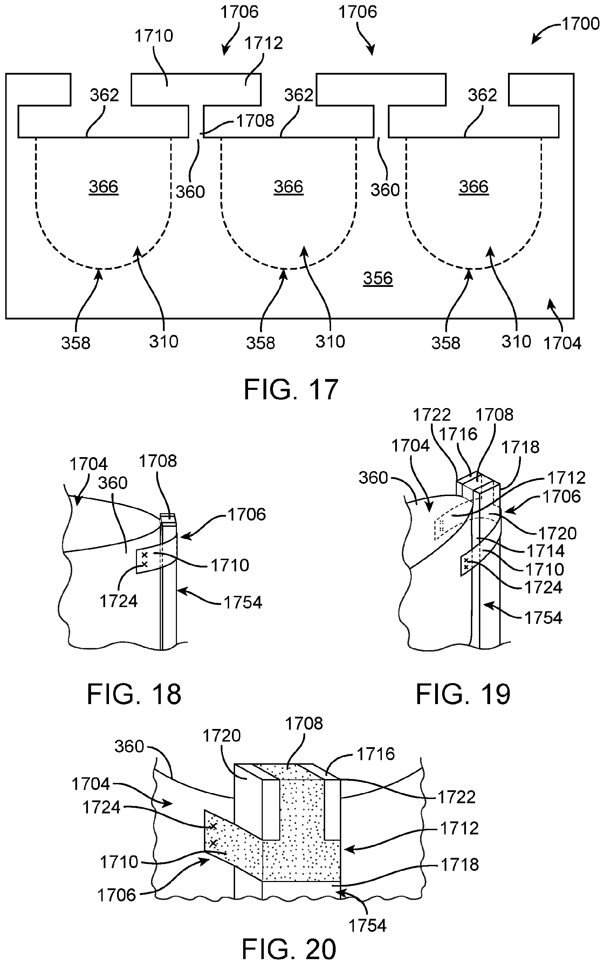

FIG. 17 is a laid flat plan view of a tissue coupon 1700 illustrating a prosthetic valve 1704 in accordance with another embodiment. FIG. 18 is side view of commissure tab 1706 of prosthetic valve 1704 of FIG. 17 attached to a trident post 1754 in accordance with one embodiment. FIG. 19 is an inner perspective view of commissure tab 1706 of prosthetic valve 1704 of FIG. 17 attached to trident post 1754 of FIG. 18 in accordance with one embodiment. FIG. 20 is an outer perspective view of commissure tab 1706 of prosthetic valve 1704 of FIG. 17 attached to trident post 1754 of FIG. 18 in accordance with one embodiment.

Tissue coupon 1700 of FIG. 17 corresponds to prosthetic valve 1704 cut longitudinally at a commissure 360. It is to be understood that tissue coupon 1700 is set forth in FIG. 17 to facilitate understanding of the fabrication of prosthetic valve 1704 and commissure tabs 1706 but that prosthetic valve 1704 including commissure tabs 1706 are fabricated from a cylindrical shaped piece of material and tissue coupon 1700 would not exist during actual fabrication.

Prosthetic valve 1704 of FIGS. 17-20 is similar to prosthetic valve 304 of FIGS. 3-6 and used with a valve prosthesis similar to valve prosthesis 300. Only the significant differences between prosthetic valve 1704 of FIGS. 17-20 and prosthetic valve 304 of FIGS. 3-6 are discussed below.

Referring now to FIGS. 17-20 together, tissue coupon 1700 includes T shaped commissure tabs 1706 extending from commissures 360. More particularly, commissure tabs 1706 include a main body 1708, a first ear 1710, and a second ear 1712. Main body 1708 extends from commissures 360. First ear 1710 extends perpendicularly from the end of main body 1708. Second ear 1712 extending perpendicularly from the end of main body 1708 in a direction opposite first ear 1710.

Paying particular attention now to FIGS. 18-20, trident post 1754 is a solid rectangular member in contrast to trident posts 354 of FIGS. 3, 6 that include a slot 384. Trident post 1754 includes an inner surface 1714, a distal end surface 1716, an outer surface 1718, and side surface 1720, 1722. Inner surface 1714 is radially inward of outer surface 1718 and side surfaces 1720, 1722 extend radially between inner surface 1714 and outer surface 1718.

Main body 1708 extends longitudinally and distally along inner surface 1714, radially outward along distal end surface 1716, and longitudinally and proximally along outer surface 1718 of trident post 1754. First ear 1710 and second ear 1712 extend from outer surface 1718 of trident post 1754 radially inward and along side surfaces 1720, 1722 of trident post 1754 and are attached to commissure 360 with tab stitching 1724. In other words, ears 1710, 1712 wrap around side surfaces 1720, 1722 of trident post 1754 to be attached to commissure 360 with tab stitching 1724. In this manner, commissure tabs 1706 wrap around and are attached to trident posts 1754.

Commissure tabs 1706 provide a radially extending attachment to trident posts 1754. By decoupling direct attachment of commissures 360 to trident posts 1754, commissure tabs 1706 reduce stress applied to prosthetic valve 1704.

Although trident post 1754 is a solid rectangular member in accordance with this embodiment, in other embodiments, trident post 1754 can have other shapes. For example, trident post 1754 is a solid cylindrical member in one embodiment and commissure tabs 1706 wrap around and are attached to the solid cylindrical member in a similar manner as that described above.

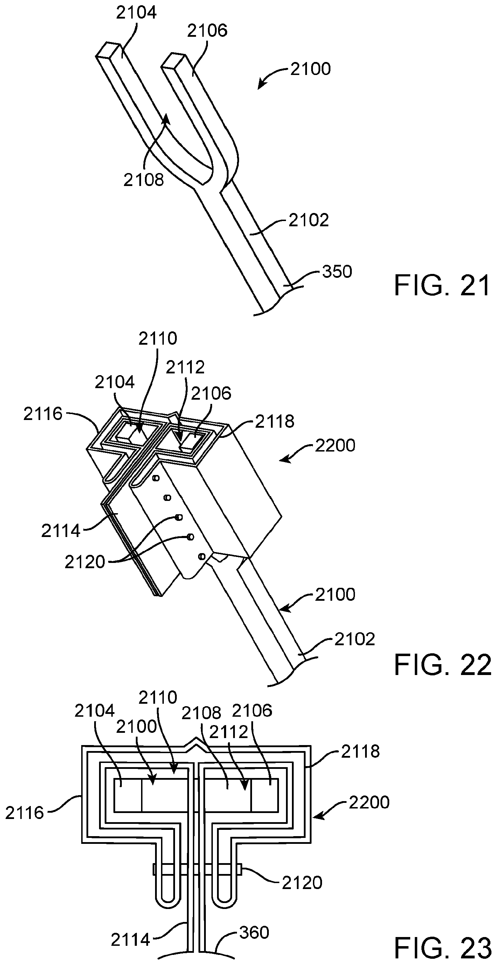

FIG. 21 is a perspective view of a Y-shaped trident post 2100 in accordance with one embodiment. FIG. 22 is a perspective view of a commissure tab 2200 attached to trident post 2100 of FIG. 21 in accordance with one embodiment. FIG. 23 is a top plan view of commissure tab 2200 attached to trident post 2100 of FIG. 22 in accordance with one embodiment.

Referring now to FIGS. 3, 6, 21-23 together, instead of trident posts 354 of valve prosthesis 300 of FIGS. 3, 6, a similar valve prosthesis is formed with trident posts 2100 having a prosthetic valve similar to prosthetic valve 304 but including commissure tabs 2200. Trident post 2100 is Y-shaped and includes a main trunk 2102 attached to an inferior crown 350 and extending distally therefrom. At a distal end of main trunk 2102, trident post 2100 includes a first branch 2104 and a second branch 2106 extending distally from main trunk 2102. An open space 2108 exists between branches 2104, 2106, space 2108 being open and unsealed in the distal direction.

Commissure tab 2200 includes a first pocket 2110 and a second pocket 2112 into which branches 2104, 2106 are positioned. More particularly, commissure tab 2200 includes a radial extending body 2114, a first ear 2116, and a second ear 2118. First ear 2116 and second ear 2118 extend from body 2114, invert back inwards and wrap around branches 2104, 2106, and are attached to body 2114 with tab stitching 2120. Body 2114 along with ears 2116, 2118 define pockets 2110, 2112, respectively.

To assemble commissure tab 2200 to trident post 2100, in one embodiment, commissure tab 2200 is fabricated as discussed above to have pockets 2110, 2112. Then branches 2104, 2106 of trident post 2100 are inserted into pockets 2110, 2112. By preassembling commissure tabs 2200 and having the open design of trident posts 2100, stitching of commissure tabs 2200 to trident post 2100 is avoided and manufacturing is simplified.

Further, by inverting and sewing ears 2116, 2118 to body 2114, commissure tab 2200 is thickened and thus strengthened. In addition, by decoupling direct attachment of commissures 360 to trident posts 2100, commissure tabs 2200 reduce stress applied to the prosthetic valve. Accordingly, commissure tabs 2200 provides a robust connection to commissures 360 while at the same time reduce stress on commissures 360.

FIG. 24 is a side view of a valve prosthesis 300A in accordance with another embodiment. Valve prosthesis 300A of FIG. 24 is similar to valve prosthesis 300 of FIG. 6 except that valve prosthesis 300A of FIG. 24 has trident posts 1154 that are solid members in contrast trident posts 354 of FIGS. 3, 6 that include a slot 384.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

Further disclosed herein is the subject-matter of the following clauses:
1. A valve prosthesis comprising:
   a stent structure;
   a one-piece molded valve coupled to the stent structure, the one-piece molded valve comprising commissures; and
   reinforcement members applied to the commissures.
2. The valve prosthesis of Clause 1 wherein the reinforcement members are rectangular strips of material.
3. The valve prosthesis of Clause 2 wherein the reinforcement members comprise a first material and the one-piece molded valve comprises a second material, wherein the first material and the second material are the same.
4. The valve prosthesis of Clause 1 or of any of the preceding Clauses, wherein:
   the stent structure comprises trident posts, further comprising commissure stitching attaching the commissures including the reinforcement members to the trident posts, the commissure stitching passing through both the commissures and the reinforcement members.
5. A valve prosthesis comprising:
   a stent structure; and
   a one-piece molded valve coupled to the stent structure, the one-piece molded valve comprising commissures and commissure cuts in the commissures.
6. The valve prosthesis of Clause 5 further comprising commissure tabs coupled to the commissures within the commissure cuts.
7. The valve prosthesis of Clause 6 wherein the commissure tabs are strips of material that are folded, inserted into the commissure cuts, and sewn to the commissures with tab stitching.
8. The valve prosthesis of Clause 7 wherein each commissure tab includes a first tab ear and a second tab ear that protrude radially outward from a center of the commissure tab, further comprising:
   commissure stitching attaching the tab ears to trident posts of the stent structure.
9. The valve prosthesis of Clause 5 or of any of Clauses 5-8, wherein the commissures are folded back at the commissure cuts to form commissure reinforcement layers.
10. The valve prosthesis of Clause 9 further comprising:
   commissure tabs inside of the commissure cuts;
   reinforcement stitching attaching the commissure reinforcement layers to the commissures and the commissure tabs; and tab stitching attaching the commissures to the commissure tabs.
11. A valve prosthesis comprising:
   a stent structure;
   a one-piece molded valve coupled to the stent structure, the one-piece molded valve comprising:
      commissures;
      commissure tabs extending from the commissures, each commissure tab comprising:
         a main body;
         a first ear attached to the main body; and
         a second ear attached to the main body.
12. The valve prosthesis of Clause 11, wherein the main body extends radially from the commissures.
13. The valve prosthesis of Clause 12 wherein the stent structure comprises trident bars, further comprising:
   tab stitching attaching the first ears and the second ears to the trident bars.
14. The valve prosthesis of Clause 13 wherein the prosthetic valve has a cylindrical shape.
15. The valve prosthesis of Clause 11 or of any of Clauses 11-14, wherein the main body extends distally from the commissures, the first ear extending perpendicularly from the main body, and the second ear extending perpendicularly from the main body and in a direction opposite the first ear.
16. The valve prosthesis of Clause 15 wherein the stent structure comprises a trident post, the commissure tab wrapping around and being attached to the trident post.
17. The valve prosthesis of Clause 16 wherein the trident post comprises:
   an inner surface;
   an outer surface;
   a distal end surface;
   a first side surface; and
   a second side surface,

   wherein the main body extends longitudinally and distally along the inner surface, radially outward along the distal end surface, and longitudinally and proximally along the outer surface, the first ear and the second ear wrapping around the side surfaces; and
   tab stitching attaching the first ear and the second ear to the commissure.
18. The valve prosthesis of Clause 11 or of any of Clauses 11-17,
   the stent structure comprising a trident post having a Y-shape, the trident post comprising:
   a main trunk;
   a first branch extending distally from the main trunk; and
   a second branch extending distally from the main trunk.
19. The valve prosthesis of Clause 18 wherein the stent structure comprises an outflow portion comprising an outflow portion crown ring comprising:
   superior crowns;
   inferior crowns; and
   outflow portion struts extending between the superior crowns and the inferior crowns, the main trunk being attached to an inferior crowns of the inferior crowns.

## Claims

1. A valve prosthesis comprising:
a stent structure;
a one-piece molded valve coupled to the stent structure, the one-piece molded valve comprising commissures; and
reinforcement members applied to the commissures.

2. The valve prosthesis of Claim 1 wherein the reinforcement members are rectangular strips of material, wherein optionally the reinforcement members comprise a first material and the one-piece molded valve comprises a second material, wherein the first material and the second material are the same.

3. The valve prosthesis of any of the preceding Claims, wherein:
the stent structure comprises trident posts, further comprising commissure stitching attaching the commissures including the reinforcement members to the trident posts, the commissure stitching passing through both the commissures and the reinforcement members.

4. A valve prosthesis comprising:
a stent structure; and
a one-piece molded valve coupled to the stent structure, the one-piece molded valve comprising commissures and commissure cuts in the commissures.

5. The valve prosthesis of Claim 4 further comprising commissure tabs coupled to the commissures within the commissure cuts.

6. The valve prosthesis of Claim 5 wherein the commissure tabs are strips of material that are folded, inserted into the commissure cuts, and sewn to the commissures with tab stitching, wherein optionally each commissure tab includes a first tab ear and a second tab ear that protrude radially outward from a center of the commissure tab, further comprising:
commissure stitching attaching the tab ears to trident posts of the stent structure.

7. The valve prosthesis of any of Claims 4-6 wherein the commissures are folded back at the commissure cuts to form commissure reinforcement layers.

8. The valve prosthesis of Claim 7 further comprising:
commissure tabs inside of the commissure cuts;
reinforcement stitching attaching the commissure reinforcement layers to the commissures and the commissure tabs; and tab stitching attaching the commissures to the commissure tabs.

9. A valve prosthesis comprising:
a stent structure;
a one-piece molded valve coupled to the stent structure, the one-piece molded valve comprising:
commissures;
commissure tabs extending from the commissures, each commissure tab comprising:
a main body;
a first ear attached to the main body; and
a second ear attached to the main body.

10. The valve prosthesis of Claim 9, wherein the main body extends radially from the commissures.

11. The valve prosthesis of Claim 10 wherein the stent structure comprises trident bars, further comprising:
tab stitching attaching the first ears and the second ears to the trident bars, wherein optionally the prosthetic valve has a cylindrical shape.

12. The valve prosthesis of any of Claims 9-11, wherein the main body extends distally from the commissures, the first ear extending perpendicularly from the main body, and the second ear extending perpendicularly from the main body and in a direction opposite the first ear.

13. The valve prosthesis of Claim 12 wherein the stent structure comprises a trident post, the commissure tab wrapping around and being attached to the trident post, wherein optionally the trident post comprises:
an inner surface;
an outer surface;
a distal end surface;
a first side surface; and
a second side surface,
wherein the main body extends longitudinally and distally along the inner surface, radially outward along the distal end surface, and longitudinally and proximally along the outer surface, the first ear and the second ear wrapping around the side surfaces; and
tab stitching attaching the first ear and the second ear to the commissure.

14. The valve prosthesis of any of Claims 9-13,
the stent structure comprising a trident post having a Y-shape, the trident post comprising:
a main trunk;
a first branch extending distally from the main trunk; and
a second branch extending distally from the main trunk.

15. The valve prosthesis of Claim 14 wherein the stent structure comprises an outflow portion comprising an outflow portion crown ring comprising:
superior crowns;
inferior crowns; and
outflow portion struts extending between the superior crowns and the inferior crowns, the main trunk being attached to an inferior crowns of the inferior crowns.
